# EUROPÄISCHE PATENTANMELDUNG

(11) **EP 2 893 888 A1**
(43) Veröffentlichungstag der Anmeldung: **15.07.2015**
(21) Anmeldenummer: 14199789.0
(22) Anmeldetag: 22.12.2014
(51) Int. Cl.: A61B 17/56, A61M 5/28, B65D 1/02, A61J 1/20

(54) **Ampullensystem mit medizinischer Flüssigkeit und Kappe mit Filtereinrichtung**

(30) Priorität: 10.01.2014 DE 102014200286
(71) Anmelder: Heraeus Medical GmbH, 61273 Wehrheim (DE)
(72) Erfinder: Wüst, Edgar, 34 Groß-Umstadt (DE)
(74) Vertreter: Panten, Kirsten

(57) **Zusammenfassung**

Die Erfindung betrifft ein Ampullensystem aufweisend eine Ampulle (1) aus Glas, wobei die Ampulle (1) einen Innenraum umschließt und einen Ampullenkörper (4) und einen abbrechbaren Ampullenkopf (5) aufweist, wobei der Ampullenkopf (5) derart vom Ampullenkörper (4) abbrechbar ist, dass sich der Innenraum durch Abbrechen des Ampullenkopfs (5) öffnet, und wobei in dem Innenraum der Ampulle (1) eine Flüssigkeit (7) für eine medizinische Anwendung oder zur Herstellung einer Mischung für eine medizinischen Anwendung enthalten ist, wobei das Ampullensystem eine Kappe (2) aufweist, die derart auf den Ampullenkörper (4) aufsteckbar ist, dass die Öffnung des Innenraums bei abgebrochenem Ampullenkopf (5) durch die Kappe (2) abgedeckt ist, und die Kappe (2) eine Filtereinrichtung (3) aufweist, wobei die Filtereinrichtung (3) zumindest ein Sieb (3) und/oder zumindest ein Filter ist und die Flüssigkeit (7) durch die Filtereinrichtung (3) aus dem Innenraum aus der Ampulle (1) ausgießbar ist.

Die Erfindung betrifft auch ein Verfahren zur Verwendung eines solchen Ampullensystems, bei dem der Ampullenkopf (5) vom Ampullenkörper (4) abgebrochen und dadurch der Innenraum der Ampulle (1) geöffnet wird, anschließend die Kappe (2) über die Öffnung der geöffneten Ampulle (1) auf die Ampulle (1) gesteckt wird und danach die Flüssigkeit (7) aus dem Innenraum der Ampulle (1) durch die Filtereinrichtung (3) gegossen wird.

## Beschreibung

Die Erfindung betrifft ein Ampullensystem aufweisend eine Ampulle aus Glas, wobei die Ampulle einen Innenraum umschließt und einen Ampullenkörper und einen abbrechbaren Ampullenkopf aufweist, wobei der Ampullenkopf derart vom Ampullenkörper abbrechbar ist, dass sich der Innenraum durch Abbrechen des Ampullenkopfs öffnet, und wobei in dem Innenraum der Ampulle eine Flüssigkeit für eine medizinische Anwendung oder zur Herstellung einer Mischung für eine medizinischen Anwendung enthalten ist sowie ein Verfahren zur Verwendung einer solchen Ampulle.

Ampullensysteme werden in der Medizin zur sterilen und sichereren Aufbewahrung von Flüssigkeiten für die medizinische Anwendung verwendet. Unter anderem kommen solche Ampullen im medizinischen Bereich bei der Aufbewahrung einer Ausgangskomponente eines Knochenzements zur Anwendung, insbesondere bei der Anwendung zur Aufbewahrung eines flüssigen Methylmethacrylats als Monomer zur Bildung eines Knochenzements aus Polymethylmethacrylat (PMMA). In der Medizin werden solche PMMA-Knochenzemente zur dauerhaften mechanischen Fixierung von Totalgelenkendoprothesen eingesetzt. Die dazu verwendeten Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Komponenten der Redoxinitiatorsysteme zusammenwirken. Deshalb werden die Komponenten des Redoxinitiatorsystems in den separaten Behältnissen so angeordnet, dass diese keine radikalische Polymerisation auslösen können. Erst bei Vermischung der beiden Komponenten zu einem Zementteig reagieren die zuvor getrennten Komponenten des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet. Die Vermischung der Komponenten erfolgt bei vielen Operationen (OP) in einer Schale, in der die beiden Komponenten eingefüllt und dann anschließend manuell gemischt werden. Das Monomer wird dabei in flüssiger Form einer Ampulle aus Glas entnommen, die zuvor aufgebrochen wurde.

Aus dem Stand der Technik sind Vakuumzementiersysteme bekannt, bei denen solche Ampullen zum Einsatz kommen. Beispielsweise offenbart die gattungsgemäße DE 10 2010 026 497 A1 ein Vakuumzementiersystem, bei dem ein Ampullenkopf abgebrochen und dadurch eine Ampulle geöffnet werden kann. Mit Hilfe eines Unterdrucks wird die Flüssigkeit aus der Ampulle beziehungsweise aus einem Lagerbehälter für die Ampulle durch eine Leitung in ein Zementpulver oder eine Zementpaste gezogen. In dieser Leitung kann ein Sieb angeordnet sein, um Splitter der Ampulle zurückzuhalten.

Die EP 0 926 500 A2 offenbart eine Vorrichtung zur Entnahme einer Flüssigkeit aus einer Glasampulle, die dazu geeignet ist, den Ampullenkopf abzuscheren und die Flüssigkeit durch eine Kanüle, in der ein Filter angeordnet sein kann, aus der geöffneten Ampulle zu entnehmen. Ein weiterer solcher komplexer Aufbau zum Öffnen und Entleeren einer Ampulle ist aus der WO 99/37256 A1 bekannt.

Für viele Anwendungen sind solche Aufbauten zu komplex und aufwendig. Häufig werden die Ausgangskomponenten von Knochenzementen einfach direkt vom Anwender in der vom Anwender gewünschten Menge in einer Schale gemischt und mit einem Spachtel bei der Operation aufgetragen. Das Monomer soll aber dazu noch immer aus Glasampullen entnommen werden können. Hierbei kann es vorteilhaft sein, dass der Anwender nicht den gesamten Inhalt einer Ampulle verbraucht, sondern eine gewünschte Menge des Monomers zugeben kann, um eine vom Anwender bereitgestellte Menge des Zementpulvers oder der Zementpaste mit dem Monomer zu vermischen. Auch bei dieser Anwendung kommen solche Glasampullen zum Einsatz, wobei dann bei der Herstellung des Knochenzements die Gefahr besteht, dass Glassplitter, die beim Öffnen der Ampulle entstehen, mit der Flüssigkeit in das Knochenzementgemischt gelangen. Solche Glassplitter können dazu führen, dass der Knochenzement geschwächt wird. Ebenso können sie sich nachteilig auf den Heilungsprozess des Patienten auswirken.

Die Aufgabe der Erfindung besteht also darin, die Nachteile des Stands der Technik zu überwinden. Insbesondere soll ein Ampullensystem und ein Verfahren zu dessen Anwendung entwickelt werden, bei der Glasampullen ohne aufwendigen Aufbau einsetzbar sind, ohne dass Glassplitter die enthaltene medizinische Flüssigkeit beeinträchtigen. Zudem soll eine beliebige Flüssigkeitsmenge verwendet werden können, um diese im medizinischen Einsatz zu verwenden, ohne dass Glassplitter die Flüssigkeit verunreinigen und ohne dass der Aufbau zu aufwendig wird. Der Aufbau soll aber gleichzeitig so einfach wie möglich zu bedienen sein. Dabei gilt es zu beachten, dass bei der Anwendung medizinischer Flüssigkeiten im OP-Bereich häufig eine Sterilisation aller mit dem Patienten oder den Behandlungsmitteln in Kontakt kommenden Teile sicher und zuverlässig ermöglicht werden muss. Die Anwendung des erfindungsgemäßen Verfahrens und das Ampullensystem sollen möglichst wenige Möglichkeiten zu Fehlanwendungen bieten. Zudem soll das Ampullensystem möglichst kostengünstig in der Herstellung sein, da die Ampullensysteme nach einmaliger Anwendung aus Gründen der Hygiene meist entsorgt werden.

Die Aufgaben der Erfindung werden gelöst durch ein Ampullensystem aufweisend eine Ampulle aus Glas, wobei die Ampulle einen Innenraum umschließt und einen Ampullenkörper und einen abbrechbaren Ampullenkopf aufweist, wobei der Ampullenkopf derart vom Ampullenkörper abbrechbar ist, dass sich der Innenraum durch Abbrechen des Ampullenkopfs öffnet, und wobei in dem Innenraum der Ampulle eine Flüssigkeit für eine medizinische Anwendung oder zur Herstellung einer Mischung für eine medizinischen Anwendung enthalten ist, wobei das Ampullensystem eine Kappe aufweist, die derart auf den Ampullenkörper aufsteckbar ist, dass die Öffnung des Innenraums bei abgebrochenem Ampullenkopf durch die Kappe abgedeckt ist, und die Kappe eine Filtereinrichtung aufweist, wobei die Filtereinrichtung zumindest ein Sieb und/oder zumindest ein Filter ist und die Flüssigkeit durch die Filtereinrichtung aus dem Innenraum aus der Ampulle ausgießbar ist.

Bevorzugt ist die Flüssigkeit ein Methylmethacrylat. Besonders bevorzugt wird das Methylmethacrylat zur Herstellung eines Polymethylmethacrylat-Knochenzements (PMMA-Knochenzements) verwendet.

Als Glas für die Ampulle kommen übliche Gläser auf Siliziumdioxid-Basis in Frage aber auch andere Materialien. Grundsätzlich sind alle Gläser, die nicht mit dem Inhalt chemisch reagieren und die ohne größere Probleme aufzubrechen sind, zur Verwendung für die Glasampullen geeignet.

Es kann erfindungsgemäß besonders bevorzugt vorgesehen sein, dass die Flüssigkeit ausschließlich durch die Filtereinrichtung aus dem Innenraum aus der Ampulle ausgießbar ist.

Die Verwendung mehrerer in Bezug auf die Flussrichtung der Flüssigkeit hintereinander angeordneter Siebe und/oder Filter hat den Vorteil, dass Glaspartikel unterschiedlicher Größe in unterschiedlichen Sieben und/oder Filtern abgefangen werden und dadurch der freie Querschnitt zum Durchfließen der Flüssigkeit größer bleibt, als wenn nur ein Filter oder Sieb verwendet wird.

Dennoch ist die Verwendung nur eines Siebs erfindungsgemäß bevorzugt, da hierdurch der Aufbau vereinfacht wird und beim Aufbrechen der Ampullen meist nur wenige Glassplitter entstehen, die zurückgehalten werden müssen.

Bei erfindungsgemäßen Ampullensystemen kann vorgesehen sein, dass die Kappe vollumfänglich an dem Ampullenkörper anliegt, wenn sie auf die Ampulle aufgesteckt ist und der Ampullenkopf abgebrochen ist.

Es kann erfindungsgemäß bevorzugt auch vorgesehen sein, dass die Kappe in Presspassung und/oder fluiddicht an dem Ampullenkörper anliegt, wenn sie auf die Ampulle aufgesteckt ist und der Ampullenkopf abgebrochen ist.

Hierdurch kann sichergestellt werden, dass die Flüssigkeit aus dem Innenraum der Ampulle nur durch die Filtereinrichtung ausgegossen werden kann und nicht an der Kappe vorbei.

Mit einer besonders bevorzugten Ausführung der Erfindung wird vorgeschlagen, dass das Ampullensystem aus der Ampulle mit der Flüssigkeit und der Kappe besteht.

Außer der Ampulle mit der Flüssigkeit und der Kappe hat das Ampullensystem dann keine weiteren Teile. Das Ampullensystem ist mit diesen wenigen Teilen zum manuellen Mischen eines Knochenzements oder zum individuellen Verwenden der Flüssigkeit ausreichend und daher einfach und kostengünstig im Aufbau.

Es kann erfindungsgemäß vorgesehen sein, dass die Kappe an dem dem Ampullenkopf gegenüberliegenden Ende des Ampullenkörpers aufgesteckt ist oder lösbar befestigt ist.

Hierdurch kann das Ampullensystem als Ganzes sterilisiert werden, ohne dass die Ampulle oder die Kappe als Einzelteile einzeln behandelt oder zumindest einzeln in die Vorrichtung zum Sterilisieren des Ampullensystems gelegt werden müssten. Die Kappe nimmt diese Position also ein bevor der Ampullenkopf vom Ampullenkörper abgebrochen ist beziehungsweise wird.

Eine Ausführungsform, bei der mögliche Glassplitter besonders zuverlässig zurückgehalten werden, ergibt sich, wenn erfindungsgemäß vorgesehen ist, dass die, bei abgebrochenem Ampullenkopf, im aufgesteckten Zustand der Kappe, der Öffnung des Innenraums zugewandte Seite der Kappe mit der anderen Seite der Kappe, die der Umgebung des Ampullensystems zugewandt ist, nur durch die Filtereinrichtung verbunden ist und ansonsten getrennt ist.

Hierdurch wird sichergestellt beziehungsweise klargestellt, dass keine weiteren Öffnungen beziehungsweise Durchführungen in der Kappe vorhanden sein sollen, durch die die Flüssigkeit mit möglicherweise enthaltenen Glassplittern durch die Kappe fließen kann. Hierdurch wird wiederum sichergestellt, dass in der Flüssigkeit enthaltene Glassplitter oder zumindest solche ab einer gewissen Größe vollständig aus der Flüssigkeit entfernt und durch das Sieb beziehungsweise den Filter entfernt werden.

Eine besonders einfach und kostengünstig zu realisierende Ausführungsform der Erfindung kann dadurch erreicht werden, dass die Kappe mit der Filtereinrichtung aus einem Kunststoff besteht, bevorzugt aus einem nicht mit Methylmethacrylat reaktionsfähigen Kunststoff, besonders bevorzugt aus Polypropylen.

Dadurch ist die Herstellung der Kappe einfach und kostengünstig zu realisieren. Beispielsweise und erfindungsgemäß bevorzugt kann die Kappe mit Hilfe eines Spritzgussverfahrens hergestellt sein. Alternativ kann auch vorgesehen sein, dass das Sieb oder die Siebe und/oder der Filter oder die Filter aus einem anderen Material (beispielsweise einem Metall oder einem Textil) bestehen und die restliche Kappe aus Kunststoff besteht. Die Filtereinrichtung(en) solcher Kappen sind dann in die restliche Kappe eingelegt oder darin befestigt.

Es kann erfindungsgemäß vorgesehen sein, dass das Sieb eine Maschenweite zwischen 0,1 mm und 0,5 mm aufweist oder die Siebe eine Maschenweite zwischen 0,1 mm und 0,5 mm aufweisen. Bevorzugt kann vorgesehen sein, dass das Sieb eine Maschenweite zwischen 0,2 mm und 0,3 mm aufweist.

Mit diesen Maschenweiten lassen sich die üblicherweise beim Abbrechen des Ampullenkopfs entstehenden Glassplitter herausfiltern beziehungsweise entfernen, wobei gleichzeitig ein möglichst hoher oder noch ausreichender Durchfluss der Flüssigkeit durch das Sieb oder die Siebe gewährleistet werden kann. Bei den angegebenen Größen der Maschenweiten kann es sich um die durchschnittliche Größe der Maschenweiten handeln.

Die Aufgaben der vorliegenden Erfindung werden auch gelöst durch ein Verfahren zur Verwendung eines solchen Ampullensystems, bei dem der Ampullenkopf vom Ampullenkörper abgebrochen und dadurch der Innenraum der Ampulle geöffnet wird, anschließend die Kappe über die Öffnung der geöffneten Ampulle auf die Ampulle gesteckt wird und danach die Flüssigkeit aus dem Innenraum der Ampulle durch die Filtereinrichtung gegossen wird.

Bevorzugt kann dabei vorgesehen sein, dass die Kappe über die Öffnung der geöffneten Ampulle auf den Ampullenkörper der Ampulle gesteckt wird.

Es kann auch vorgesehen sein, dass vor dem Abbrechen des Ampullenkopfs die Kappe auf der Ampulle steckt und beide gemeinsam sterilisiert werden und die Kappe vor dem Abbrechen des Ampullenkopfs von der Seite der Ampulle mit dem Ampullenkopf abgezogen wird oder die Kappe nach Abbrechen des Ampullenkopfs von der dem Ampullenkopf gegenüberliegenden Seite der Ampulle abgezogen wird.

Durch diese Verfahren wird sichergestellt, dass die Kappe immer gemeinsam mit der Ampulle verwendet wird und ohne Fehlbedienung nicht verloren werden kann oder gesucht werden muss. Zudem kann so auch sichergestellt werden, dass sowohl die Kappe als auch die Ampulle sterilisiert werden und nicht eines von beidem ungewollt nicht sterilisiert wird.

Alternativ dazu könnte auch vorgesehen sein, dass die Kappe über eine Verbindung, beispielsweise über einen Steg, mit dem Ampullenkörper verbunden ist. Dazu könnte eine Schlaufe, die um den Ampullenkörper herum anliegt, die Kappe über den Steg am Ampullenkörper befestigen. Die Kappe wird dann durch biegen des Stegs über die Öffnung auf den Ampullenkörper aufgesteckt.

Mit einer besonders bevorzugten Ausführungsform des Verfahrens kann vorgesehen sein, dass nach dem Ausgießen der Flüssigkeit die Flüssigkeit mit einem Pulver oder einer Paste zur Herstellung eines Zements gemischt wird, bevorzugt zur Herstellung eines Knochenzements, besonders bevorzugt die Flüssigkeit ein Methylmethacrylat ist und zur Herstellung eines PMMA-Knochenzements mit einem Pulver oder einer Paste gemischt wird.

Der Erfindung liegt die überraschende Erkenntnis zugrunde, dass es durch die Verwendung einer Kappe mit zumindest einem Sieb oder zumindest einem Filter gelingt, Glassplitter, die beim Öffnen der Glasampulle entstehen können, auf eine kostengünstige und einfache Weise zurückzuhalten. Dadurch, dass die Kappe mit der Filtereinrichtung auch auf das gegenüberliegende Ende der Ampulle aufgesteckt werden kann, können beide gemeinsam sterilisiert werden, ohne dass eines der Teile (wahrscheinlich meist die Kappe) vergessen wird. und unsterilisiert bleibt. Bei der Anwendung eines erfindungsgemäßen Ampullensystems kann die Ampulle geöffnet werden und anschließend die Kappe mit der Filtereinrichtung einfach über die entstandene Öffnung gestülpt werden. Danach ist die geöffnete Ampulle einsatzbereit.

Im Folgenden werden weitere Ausführungsbeispiele der Erfindung anhand von fünf schematisch dargestellten Figuren erläutert, ohne jedoch dabei die Erfindung zu beschränken. Dabei zeigt:
Figur 1: eine schematische perspektivische Ansicht eines erfindungsgemäßen Ampullensystems;
Figur 2: eine schematische Querschnittansicht eines weiteren erfindungsgemäßen Ampullensystems im Ausgangszustand;
Figur 3: eine schematische Querschnittansicht des Ampullensystems nach Figur 2 mit geöffneter Ampulle;
Figur 4: eine schematische Querschnittansicht des Ampullensystems nach Figur 2 mit geöffneter Ampulle und über die Öffnung aufgesteckter Kappe; und
Figur 5: eine schematische Querschnittansicht des Ampullensystems nach Figur 4 bei dem die Flüssigkeit aus dem Innenraum aus der Ampulle ausgegossen wird.

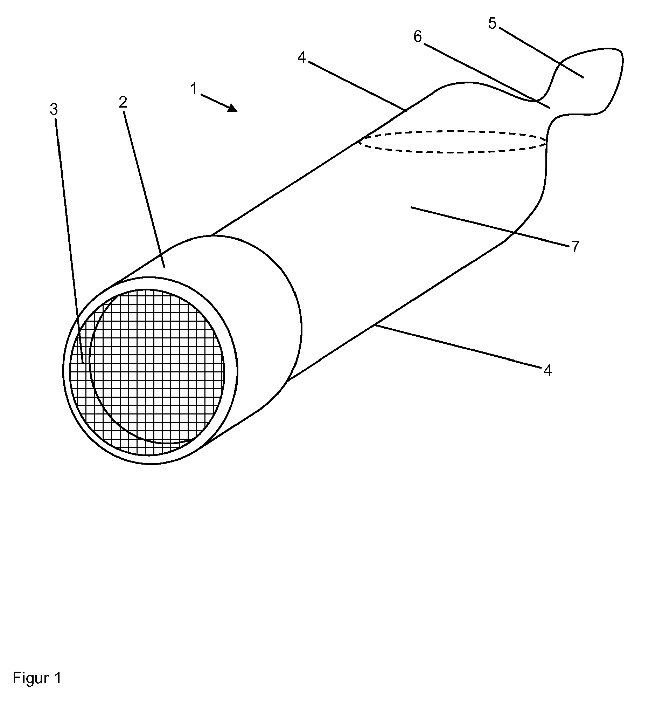

Figur 1 zeigt eine schematische perspektivische Ansicht eines erfindungsgemäßen Ampullensystems mit einer Ampulle 1 aus Glas und einer Kappe 2 aus Kunststoff. Die Kappe 2 ist ein, durch ein Sieb 3 einseitig geschlossenes Rohr aus Kunststoff. Das Sieb 3, das ebenfalls aus Kunststoff bestehen kann, hat eine durchschnittliche Maschenweite von 0,25 mm mit einer Standardabweichung von +/- 0,05 mm.

Die Ampulle 1 besteht aus einem Ampullenkörper 4 und einem Ampullenkopf 5, die über einen Ampullenhals 6 miteinander verbunden sind. Die Ampulle 1 kann geöffnet werden, indem der Ampullenkopf 5 vom Ampullenkörper 4 abgebrochen wird. Dabei bricht die Ampulle 1 an der dafür vorgesehenen Sollbruchstelle am Ampullenhals 6. Im Inneren der Ampulle 1 befindet sich eine Flüssigkeit 7 zur Verwendung bei einer medizinischen Anwendung. Die Ampulle 1 ist nicht vollständig mit der Flüssigkeit 7 gefüllt. Es befindet sich also ein Gasüberstand (in Figur 1 oben) in der Ampulle 1. In Figur 1 ist die Grenzfläche der Flüssigkeit 7 zum Gasüberstand als gestrichelte Linie dargestellt. Beispielsweise können Impfstoffe, Kalziumlösungen oder eine flüssige Ausgangskomponente für einen Knochenzement als Flüssigkeit 7 in der Ampulle 1 enthalten sein.

Die Kappe 2 steckt auf der zum Ampullenkopf 5 gegenüberliegenden Seite der Ampulle 1. Der Boden der Ampulle 1 ist durch die Maschen des Siebs 3 in Figur 1 erkennbar. Der Innendurchmesser der Kappe 2 ist so gewählt, dass er von beiden Seiten auf die Ampulle 1 aufgesteckt werden kann und dort durch Presspassung ansitzt. Verbleibende Spalten sind vorzugsweise kleiner als oder höchstens genauso groß wie die Maschenweite des Siebs 3. Der Ampullenkörper 4 ist zylindrisch geformt mit abnehmendem Durchmesser am Ampullenhals 6 und mit abgerundet geschlossenem Ende am Ampullenboden. Die Wandstärke der Ampulle 1 ist gleichmäßig (im Rahmen üblicher Produktionsabweichungen) und kann im Bereich des Ampullenhalses 6 dünner sein, um ein Brechen der Ampulle 1 am Ampullenhals 6 zu begünstigen.

Bei einer Anwendung des Ampullensystems zur Herstellung von PMMA-Knochenzement wird das Ampullensystem, so wie es in Figur 1 gezeigt ist, zum Sterilisieren in eine Sterilisationsvorrichtung beziehungsweise -kammer gelegt. Mit einem sterilisierenden Gas wird sowohl die Ampulle 1 als auch die Kappe 2 mit dem Sieb 3 sterilisiert. Auch bei einer anderen Anwendung kann selbstverständlich eine Sterilisation erfolgen.

Nach der Sterilisation oder ohne Sterilisation wird das Ampullensystem verwendet. Der Ampullenkopf 5 wird manuell abgebrochen. Anschließend wird die Kappe 2 vom Ampullenboden abgezogen und auf der anderen Seite über die Öffnung im Ampullenhals 6 auf den Ampullenkörper 4 aufgesteckt. Der flüssige Inhalt 7 der Ampulle 1 wird durch das Sieb 3 aus der Ampulle ausgeleert. Alle Glassplitter, die beim Abbrechen des Ampullenkopfs 5 entstanden sind und in die Flüssigkeit 7 gelangt sind, werden beim Ausgießen von dem Sieb 3 zurückgehalten.

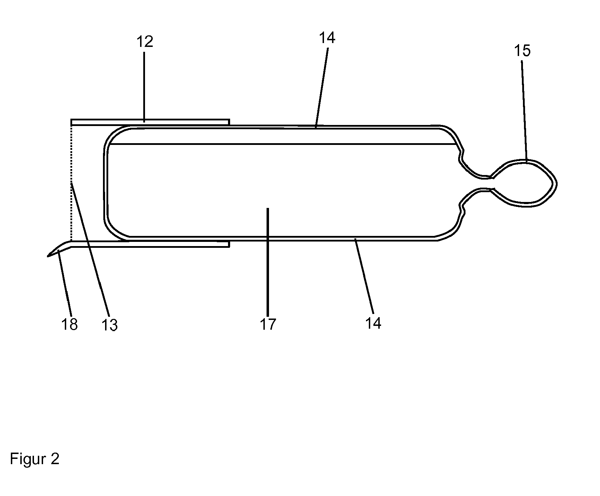

Die Figuren 2 bis 5 zeigen schematische Querschnittansichten von erfindungsgemäßen Ampullensystemen, mit denen der Ablauf eines erfindungsgemäßen Verfahrens und die Funktion des erfindungsgemäßen Ampullensystems dargestellt werden. Figur 2 zeigt eine schematische Querschnittansicht eines erfindungsgemäßen Ampullensystems im Ausgangszustand. Auf einen Ampullenboden der Ampulle ist eine Kappe 12 mit einem Sieb 13 in Form eines Gitters aufgesteckt. Die Ampulle selbst ist in einen Ampullenkörper 14 und einen Ampullenkopf 15 aufgeteilt, die einteilig miteinander verbunden sind und die einen gemeinsamen Innenraum begrenzen, in dem ein flüssiges Methylmethacrylat 17 als Flüssigkeit 17 für medizinische Zwecke, nämlich zur Herstellung eines PMMA-Knochenzements enthalten ist. Die Oberfläche der Flüssigkeit 17 ist als waagerechte Linie in Figur 2 angedeutet. An der Kappe 12 ist eine Tülle 18 vorgesehen, mit der das Ausgießen der Flüssigkeit 17 erleichtert wird. Die Tülle 18 muss nicht vorgesehen sein, hat aber für das Verwenden des Ampullensystems den Vorteil, dass bei geeigneter Form der Tülle 18 ein präzises Ausgießen der Flüssigkeit 17 möglich ist, ohne dass es zu Abtropfen oder ohne dass es zu an der Außenseite der Kappe 12 herunterlaufenden Resten der Flüssigkeit 17 kommt.

In der in Figur 2 gezeigten Position wird das Ampullensystem zunächst mit einem sterilisierenden Gas sterilisiert. Dabei wird sowohl die Ampulle als auch die Kappe 12 sterilisiert. Anschließend ist das Ampullensystem für den Einsatz bei einer Operation bereit.

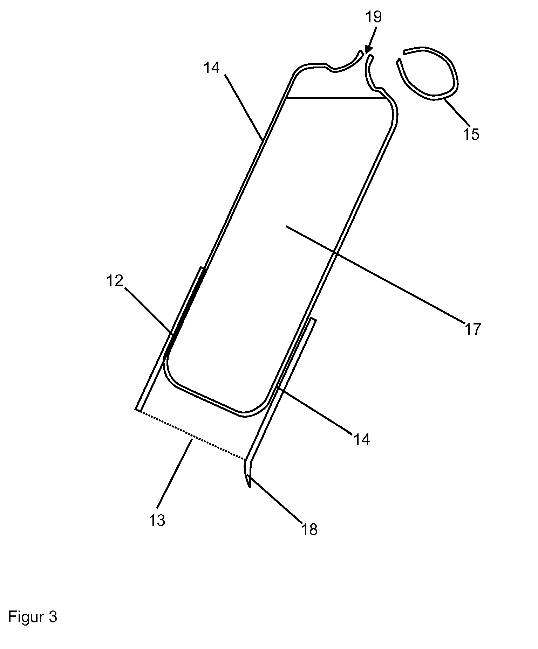

Zum Öffnen der Ampulle wird der Ampullenkopf 15 manuell vom Ampullenkörper 14 abgebrochen. Dieser Zustand ist in Figur 3 als schematische Querschnittansicht gezeigt. Die Ampulle wird dabei selbstverständlich so gehalten, dass die Flüssigkeit 17 nicht aus der Ampulle ausläuft. Die Ampulle hat dann oben eine Öffnung 19, durch die die Flüssigkeit 17 aus der Ampulle ausgegossen werden kann. Beim Abbrechen der Ampulle können sich kleine Glassplitter (nicht gezeigt) von der Bruchstelle lösen und in die Flüssigkeit 17 fallen.

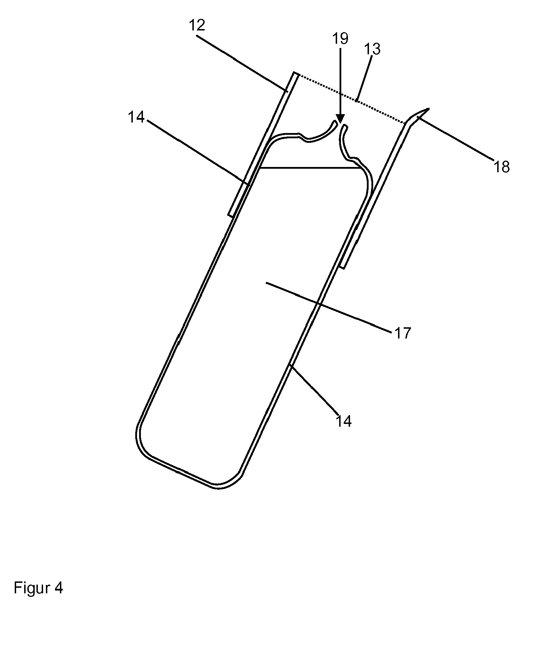

Um zu verhindern dass diese Glassplitter mit der Flüssigkeit weiterverarbeitet werden, wird anschließend die Kappe 12 auf die andere Seite der Ampulle gesteckt und zwar über die Öffnung 19. Diese Situation ist in Figur 4 gezeigt. Die Innenwandungen der Kappe 12 sind derart geformt, dass sie den Außenumfang der im Bereich des Ampullenkörpers 14, der bereichsweise zylindrisch geformt ist, umlaufend und formschlüssig abschließen. Das Material, aus dem die Kappe 12 geformt ist (vorzugsweise ein Spritzgussteil aus Kunststoff), hat die notwendige Elastizität und Verformbarkeit, dass sich die Kappe 12 ohne weiteres auf den Ampullenkörper 14 aufstecken lässt und dort durch den Presssitz der Kappe 12 auf dem Ampullenkörper 14 gehalten wird. Der einzige Durchlass der Kappe 12 und damit des Ampullensystems ist durch das Sieb 13 beziehungsweise das Gitter 13 abgedeckt. Die Flüssigkeit 17 aus der Ampulle kann nur durch das Gitter 13 beziehungsweise das Sieb 13 ausgegossen werden.

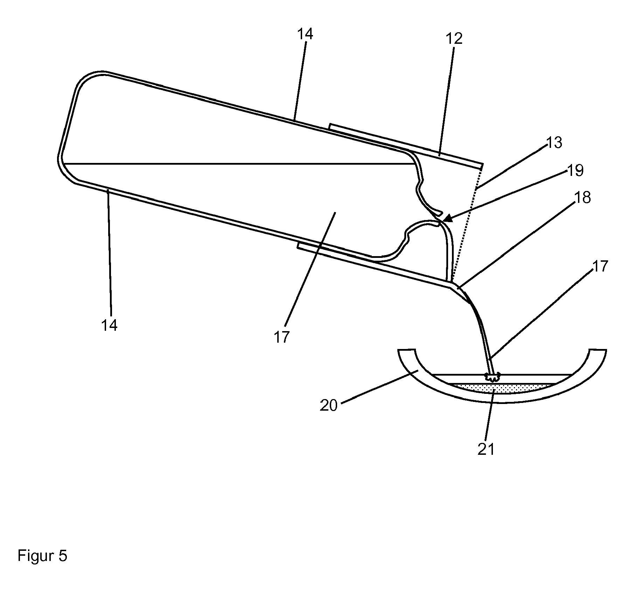

Die Situation beim Ausgießen ist in einer schematischen Darstellung als Querschnitt in Figur 5 gezeigt. Die Flüssigkeit 17 wird aus der Ampulle durch das Sieb 13 beziehungsweise das Gitter 13 über die Tülle 18 in eine Schale 20 gegossen, in der sich ein Pulver 21 als zweite Komponente des PMMA-Knochenzements befindet. Die Flüssigkeit 17 und das Pulver 21 können in der Schale 20 mit Hilfe eines Spachtels vermischt werden und der fertig angerührte Knochenzement steht zur Verwendung bereit. Das Gitter 13 beziehungsweise das Sieb 13 haben eine Maschenweite zwischen 0,2 mm und 0,3 mm und halten dementsprechend größere Glassplitter zurück.

Damit wird verhindert, dass die Glassplitter ungewollt in den Knochenzement gelangen und diesen oder dessen Anwendbarkeit beeinträchtigen.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Ampulle
- 2, 12: Kappe
- 3, 13: Sieb
- 4, 14: Ampullenkörper
- 5, 15: Ampullenkopf
- 6: Ampullenhals
- 7, 17: Flüssigkeit
- 18: Tülle
- 19: Öffnung
- 20: Schale
- 21: Pulver

## Patentansprüche

1. Ampullensystem aufweisend eine Ampulle (1) aus Glas, wobei die Ampulle (1) einen Innenraum umschließt und einen Ampullenkörper (4, 14) und einen abbrechbaren Ampullenkopf (5, 15) aufweist, wobei der Ampullenkopf (5, 15) derart vom Ampullenkörper (4, 14) abbrechbar ist, dass sich der Innenraum durch Abbrechen des Ampullenkopfs (5, 15) öffnet, und wobei in dem Innenraum der Ampulle (1) eine Flüssigkeit (7, 17) für eine medizinische Anwendung oder zur Herstellung einer Mischung für eine medizinischen Anwendung enthalten ist, **dadurch gekennzeichnet, dass**
das Ampullensystem eine Kappe (2, 12) aufweist, die derart auf den Ampullenkörper (4, 14) aufsteckbar ist, dass die Öffnung (19) des Innenraums bei abgebrochenem Ampullenkopf (5, 15) durch die Kappe (2, 12) abgedeckt ist, und die Kappe (2, 12) eine Filtereinrichtung (3, 13) aufweist, wobei die Filtereinrichtung (3, 13) zumindest ein Sieb (3, 13) und/oder zumindest ein Filter ist und die Flüssigkeit (7, 17) durch die Filtereinrichtung (3, 13) aus dem Innenraum aus der Ampulle (1) ausgießbar ist.

2. Ampullensystem nach Anspruch 1, **dadurch gekennzeichnet, dass** die Kappe (2, 12) vollumfänglich an dem Ampullenkörper (4, 14) anliegt, wenn sie auf die Ampulle (1) aufgesteckt ist und der Ampullenkopf (5, 15) abgebrochen ist.

3. Ampullensystem nach Anspruch 1 oder 2, **dadurch gekennzeichnet, dass** das Ampullensystem aus der Ampulle (1) mit der Flüssigkeit (7, 17) und der Kappe (2, 12) besteht.

4. Ampullensystem nach einem der vorangehenden Ansprüche, **dadurch gekennzeichnet, dass**
die Kappe (2, 12) an dem dem Ampullenkopf (5, 15) gegenüberliegenden Ende des Ampullenkörpers (4, 14) aufgesteckt ist oder lösbar befestigt ist.

5. Ampullensystem nach einem der vorangehenden Ansprüche, **dadurch gekennzeichnet, dass**
die, bei abgebrochenem Ampullenkopf (5, 15), im aufgesteckten Zustand der Kappe (2, 12), der Öffnung (19) des Innenraums zugewandte Seite der Kappe (2, 12) mit der anderen Seite der Kappe (2, 12), die der Umgebung des Ampullensystems zugewandt ist, nur durch die Filtereinrichtung (3, 13) verbunden ist und ansonsten getrennt ist.

6. Ampullensystem nach einem der vorangehenden Ansprüche, **dadurch gekennzeichnet, dass**
die Kappe (2, 12) mit der Filtereinrichtung (3, 13) aus einem Kunststoff besteht, bevorzugt aus einem nicht mit Methylmethacrylat reaktionsfähigen Kunststoff, besonders bevorzugt aus Polypropylen.

7. Ampullensystem nach einem der vorangehenden Ansprüche, **dadurch gekennzeichnet, dass**
das Sieb (3, 13) eine Maschenweite zwischen 0,1 mm und 0,5 mm aufweist oder die Siebe (3, 13) eine Maschenweite zwischen 0,1 mm und 0,5 mm aufweisen, bevorzugt das Sieb (3, 13) eine Maschenweite zwischen 0,2 mm und 0,3 mm aufweist.

8. Verfahren zur Verwendung eines Ampullensystems nach einem der vorangehenden Ansprüche, **dadurch gekennzeichnet, dass**
der Ampullenkopf (5, 15) vom Ampullenkörper (4, 14) abgebrochen und dadurch der Innenraum der Ampulle (1) geöffnet wird, anschließend die Kappe (2, 12) über die Öffnung (19) der geöffneten Ampulle (1) auf die Ampulle (1) gesteckt wird und danach die Flüssigkeit (7, 17) aus dem Innenraum der Ampulle (1) durch die Filtereinrichtung (3, 13) gegossen wird.

9. Verfahren nach Anspruch 8, **dadurch gekennzeichnet, dass**
vor dem Abbrechen des Ampullenkopfs (5, 15) die Kappe (2, 12) auf der Ampulle (1) steckt und beide gemeinsam sterilisiert werden und die Kappe (2, 12) vor dem Abbrechen des Ampullenkopfs (5, 15) von der Seite der Ampulle (1) mit dem Ampullenkopf (5, 15) abgezogen wird oder die Kappe (2, 12) nach Abbrechen des Ampullenkopfs (5, 15) von der dem Ampullenkopf (5, 15) gegenüberliegenden Seite der Ampulle (1) abgezogen wird.

10. Verfahren nach Anspruch 8 oder 9, **dadurch gekennzeichnet, dass**
nach dem Ausgießen der Flüssigkeit (7, 17) die Flüssigkeit (7, 17) mit einem Pulver (21) oder einer Paste zur Herstellung eines Zements gemischt wird, bevorzugt zur Herstellung eines Knochenzements, besonders bevorzugt die Flüssigkeit (7, 17) ein Methylmethacrylat ist und zur Herstellung eines PMMA-Knochenzements mit einem Pulver (21) oder einer Paste gemischt wird.
